# EUROPEAN PATENT APPLICATION

(11) **EP 4 847 479 A1**
(43) Date of publication of application: **02.12.2026**
(21) Application number: 25745239.1
(22) Date of filing: 24.01.2025
(51) Int. Cl.: C07C 17/10, C07C 17/20, C07C 19/10

(54) **METHOD FOR PRODUCING 1,1,2,3,4-PENTACHLORO-1,2,3,4,4-PENTAFLUOROBUTANE AND METHOD FOR PRODUCING 1,2,3,4-TETRACHLORO-1,1,2,3,4,4-HEXAFLUOROBUTANE**

(30) Priority: 25.01.2024 JP 2024009417
(71) Applicant: KANTO DENKA KOGYO CO., LTD., Tokyo 100-0005 (JP)
(72) Inventor: KIKUCHI, Sho, Kurashiki-shi, Okayama 712-8533 (JP); KISHI, Kazuki, Shibukawa-shi, Gunma 377-8513 (JP); ASAHINA, Kazuma, Kurashiki-shi, Okayama 712-8533 (JP); TANIDA, Tatsumi, Shibukawa-shi, Gunma 377-8513 (JP)
(74) Representative: Pfenning, Meinig & Partner mbB
(86) International application number: PCT/JP2025/002139
(87) International publication number: WO 2025/159170

(57) **Abstract**

In a method for producing 1,2,3,4-tetrachlorohexafluorobutane from 1,3-butadiene as a starting material, byproducts and the like generated by a chlorination reaction of 1,3-butadiene are converted into 1,2,3,4-tetrachlorohexafluorobutane as the valuable material to increase productivity of 1,2,3,4-tetrachlorohexafluorobutane. A method for producing 1,1,2,3,4-pentachloro-1,2,3,4,4-pentafluorobutane, the method including:
(a) a step of contacting a composition containing at least one selected from the group consisting of 1-chloro-1,3-butadiene, 1,3,4-trichloro-1-butene, and 1,1,4-trichloro-2-butene with chlorine (Cl₂) to obtain a composition containing 1,1,2,3,4-pentachlorobutane; and
(b) a step of contacting the composition containing 1,1,2,3,4-pentachlorobutane obtained in step (a) with fluorine (F₂) to obtain a composition containing 1,1,2,3,4-pentachloro-1,2,3,4,4-pentafluorobutane. In addition, a method for producing 1,2,3,4-tetrachloro-1,1,2,3,4,4-hexafluorobutane, the method including, in addition to steps (a) and (b):
(c) a step of contacting the composition containing 1,1,2,3,4-pentachloro-1,2,3,4,4-pentafluorobutane obtained in step (b) with chlorine monofluoride (ClF) to obtain a composition containing 1,2,3,4-tetrachloro-1,1,2,3,4,4-hexafluorobutane.

## Description

### TECHNICAL FIELD

The present invention relates to a method for producing 1,1,2,3,4-pentachloro-1,2,3,4,4-pentafluorobutane (hereinafter, simply referred to as 1,1,2,3,4-pentachloropentafluorobutane) and a method for producing 1,2,3,4-tetrachloro-1,1,2,3,4,4-hexafluorobutane (hereinafter, simply referred to as 1,2,3,4-tetrachlorohexafluorobutane).

### BACKGROUND ART

As a synthesis raw material and the like for hexafluoro-1,3-butadiene, which is used as an etching gas used for fine processing for semiconductors, 1,2,3,4-tetrachlorohexafluorobutane is an important compound. As a method for producing this 1,2,3,4-tetrachlorohexafluorobutane, methods described in the following patent literature are conventionally known.
(1) PTL 1 describes a method for producing 1,2,3,4-tetrachlorohexafluorobutane by allowing a reaction between one or more selected from compounds represented by CClX¹X²-CX³=CX⁴-CClX⁵X⁶, CX¹X²=X³-CClX⁴-CClX⁵X⁶, and CX¹X²=X³-CX⁴=CX⁵X⁶ (X¹ to X⁶ each independently represent a hydrogen atom or a fluorine atom) and chlorine in a liquid phase to obtain a compound represented by CClX¹X²-CClX³-CClX⁴-CClX⁵X⁶ (X¹ to X⁶ each independently represent a hydrogen atom or a fluorine atom), and then allowing a reaction between this compound represented by CClX¹X²-CClX³-CClX⁴-CClX⁵X⁶ (X¹ to X⁶ each independently represent a hydrogen atom or a fluorine atom) and fluorine in a liquid phase.
(2) Claim 1 of PTL 2 describes a method for producing 1,2,3,4-tetrachlorohexafluorobutane characterized by regulating an amount of hydrogen fluoride in a solvent used for a reaction between 1,2,3,4-tetrachlorobutane and fluorine within a range of 10 to 60 mass%.

### CITATION LIST

### PATENT LITERATURE

PTL 1: Japanese Patent Laid-Open No. 2006-342059
PTL 2: Japanese Patent No. 5274449

### SUMMARY OF INVENTION

### TECHNICAL PROBLEM

The raw material for 1,2,3,4-tetrachlorohexafluorobutane, 1,2,3,4-tetrachlorobutane, can be obtained by a reaction between 1,3-butadiene and chlorine (Cl₂), but byproducts in this reaction have not been investigated in detail, and effective use of the byproducts has not been discussed accordingly. The same applies to byproducts generated by the reaction between 1,2,3,4-tetrachlorobutane and fluorine (F₂).

An object of the present invention is to increase productivity of 1,2,3,4-tetrachlorohexafluorobutane by converting the byproducts and the like generated by the chlorination reaction of 1,3-butadiene into 1,2,3,4-tetrachlorohexafluorobutane as a valuable material in the method for producing 1,2,3,4-tetrachlorohexafluorobutane from 1,3-butadiene as the starting material.

### SOLUTION TO PROBLEM

The present invention provides the followings.
[1] A method for producing 1,1,2,3,4-pentachloro-1,2,3,4,4-pentafluorobutane, the method including:
   (a) a step of contacting a composition containing at least one selected from the group consisting of 1-chloro-1,3-butadiene, 1,3,4-trichloro-1-butene, and 1,1,4-trichloro-2-butene with chlorine (Cl₂) to obtain a composition containing 1,1,2,3,4-pentachlorobutane; and
   (b) a step of contacting the composition containing 1,1,2,3,4-pentachlorobutane obtained in step (a) with fluorine (F₂) to obtain a composition containing 1,1,2,3,4-pentachloro-1,2,3,4,4-pentafluorobutane.
[2] The method according to [1], wherein the composition containing at least one selected from the group consisting of 1-chloro-1,3-butadiene, 1,3,4-trichloro-1-butene, and 1,1,4-trichloro-2-butene in step (a) contains a product of a reaction between 1,3-butadiene and chlorine (Cl₂).
[3] The method according to [2], wherein the product of the reaction between 1,3-butadiene and chlorine (Cl₂) further contains 1,1,2,3,4-pentachlorobutane, and generating 1,1,2,3,4-pentachlorobutane by step (a) increases a concentration of 1,1,2,3,4-pentachlorobutane in a reaction solution.
[4] The method according to [2] or [3], wherein the product of the reaction between 1,3-butadiene and chlorine (Cl₂) further contains 1,1,2,4-tetrachloro-3-butene.
[5] The method according to any one of [1] to [3], wherein UV irradiation is performed in step (a).
[6] A method for producing 1,2,3,4-tetrachloro-1,1,2,3,4,4-hexafluorobutane, the method including:
   (a) a step of contacting a composition containing at least one selected from the group consisting of 1-chloro-1,3-butadiene, 1,3,4-trichloro-1-butene, and 1,1,4-trichloro-2-butene with chlorine (Cl₂) to obtain a composition containing 1,1,2,3,4-pentachlorobutane;
   (b) a step of contacting the composition containing 1,1,2,3,4-pentachlorobutane obtained in step (a) with fluorine (F₂) to obtain a composition containing 1,1,2,3,4-pentachloro-1,2,3,4,4-pentafluorobutane; and
   (c) a step of contacting the composition containing 1,1,2,3,4-pentachloro-1,2,3,4,4-pentafluorobutane obtained in step (b) with chlorine monofluoride (ClF) to obtain a composition containing 1,2,3,4-tetrachloro-1,1,2,3,4,4-hexafluorobutane.
[7] The method according to [6], wherein the composition containing at least one selected from the group consisting of 1-chloro-1,3-butadiene, 1,3,4-trichloro-1-butene, and 1,1,4-trichloro-2-butene in step (a) contains a product of a reaction between 1,3-butadiene and chlorine (Cl₂).
[8] The method according to [7], wherein the product of the reaction between 1,3-butadiene and chlorine (Cl₂) further contains 1,1,2,3,4-pentachlorobutane, and generating 1,1,2,3,4-pentachlorobutane by step (a) increases a concentration of 1,1,2,3,4-pentachlorobutane in a reaction solution.
[9] The method according to [7] or [8], wherein the product of the reaction between 1,3-butadiene and chlorine (Cl₂) further contains 1,1,2,4-tetrachloro-3-butene.
[10] The method according to any one of [6] to [8], wherein UV irradiation is performed in step (a).

### ADVANTAGEOUS EFFECTS OF INVENTION

The present invention exhibits advantageous effects, in the method for producing 1,2,3,4-tetrachlorohexafluorobutane from 1,3-butadiene as the starting material, from two viewpoints in which: the byproducts such as 1-chloro-1,3-butadiene, 1,3,4-trichloro-1-butene, 1,1,4-trichloro-2-butene, 1,1,2,4-tetrachloro-3-butene, and 1,1,2,3,4-pentachlorobutane generated in the chlorination step can be converted into 1,2,3,4-tetrachlorohexafluorobutane as a valuable material, and further, 1,2,3,4-tetrachlorohexafluorobutane can be converted into hexafluoro-1,3-butadiene, sellable as a product (a merit of improvement in productivity); and an amount of waste liquid that are conventionally treated as industrial waste, specifically as chlorine-containing organic waste liquid, can be reduced (a merit of reduction in environmental burden).

### DESCRIPTION OF EMBODIMENTS

### (Action)

The present inventors have found that, in a method of reacting 1,3-butadiene and chlorine (Cl₂) to obtain 1,2,3,4-tetrachlorobutane and reacting 1,2,3,4-tetrachlorobutane and fluorine (F₂) to produce 1,2,3,4-tetrachlorohexafluorobutane, one cause of decrease in the yield is byproducts of 1-chloro-1,3-butadiene, 1,3,4-trichloro-1-butene, 1,1,4-trichloro-2-butene, 1,1,2,4-tetrachloro-3-butene, and 1,1,2,3,4-pentachlorobutane generated in the first chlorination step, and further, products of 1,1,2,3,4-pentachloropentafluorobutane and 1,1,2,3,4,4-hexachloro-1,2,3,4-tetrafluorobutane (hereinafter, simply referred to as 1,1,2,3,4,4-hexachlorotetrafluorobutane) generated in the next fluorination step as shown in the following reaction scheme.

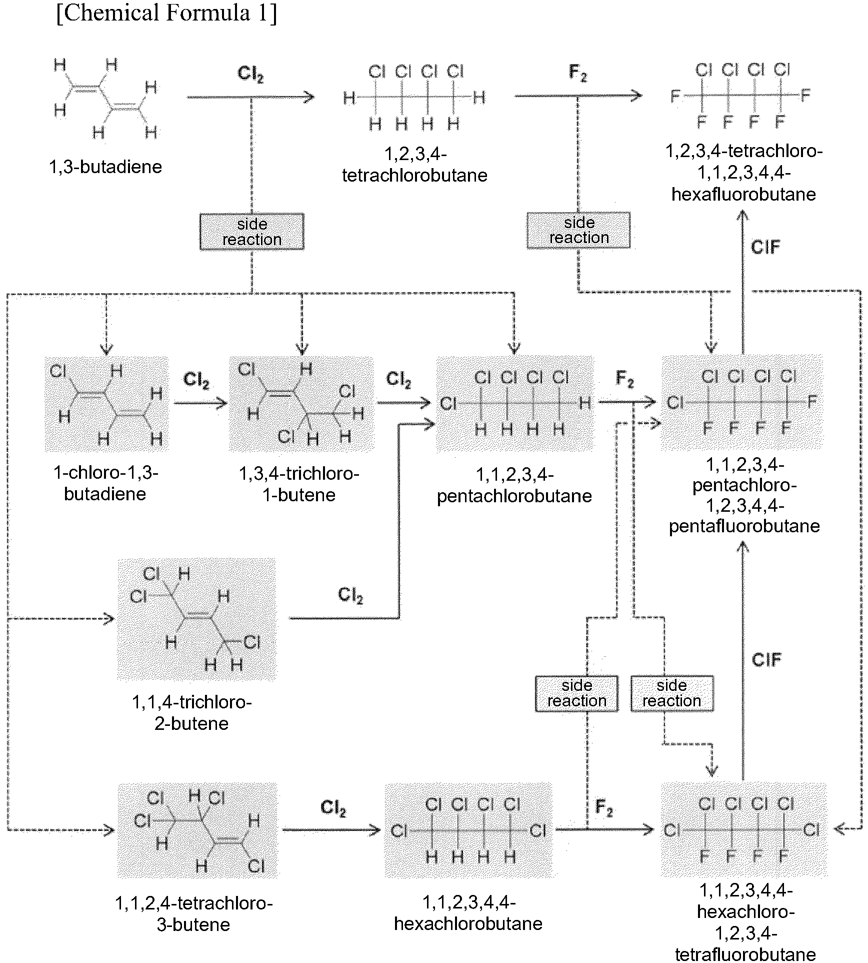

The byproduct in the fluorination step, 1,1,2,3,4-pentachloropentafluorobutane, is generated by substituting hydrogen atoms in 1,1,2,3,4-pentachlorobutane generated as a byproduct in the chlorination step with fluorine (F₂), and also generated as a byproduct from the reaction between 1,2,3,4-tetrachlorobutane and fluorine (F₂). In the reaction between 1,1,2,3,4-pentachlorobutane and fluorine (F₂), 1,1,2,3,4,4-hexachlorotetrafluorobutane is also generated in addition to the above-described 1,1,2,3,4-pentachloropentafluorobutane.

The byproduct in the chlorination step, 1,1,2,3,4-pentachlorobutane, is generated by adding one molecule of chlorine (Cl₂) to 1,3,4-trichloro-1-butene. It is considered that 1,3,4-trichloro-1-butene is generated by: adding one molecule of chlorine (Cl₂) to the starting material 1,3-butadiene, then eliminating hydrogen chloride (HCl) to generate 1-chloro-1,3-butadiene as a byproduct, and adding chlorine (Cl₂) to this 1-chloro-1,3-butadiene. In the chlorination step, 1,1,4-trichloro-2-butene, 1,1,2,4-tetrachloro-3-butene, and the like are generated, and it is considered that such various byproducts are generated by action of an ion reaction and a radical reaction.

Etching gas products used for fine processing for semiconductors are required to have high purity. Thus, in common producing methods, a starting material having purity as high as possible is used also in the process of producing 1,2,3,4-tetrachlorohexafluorobutane, which is a synthesis raw material for hexafluoro-1,3-butadiene, and distillation is performed in each of the reaction steps to remove byproducts that are other than the target product in each step. In this case, the byproducts separated by distillation are treated as industrial waste.

The byproducts, 1-chloro-1,3-butadiene, 1,3,4-trichloro-1-butene, and 1,1,4-trichloro-2-butene, generated in the first chlorination step, can be converted into 1,1,2,3,4-pentachlorobutane by reactions with chlorine gas (Cl₂). Then, 1,1,2,3,4-pentachlorobutane can be converted into 1,1,2,3,4-pentachloropentafluorobutane by a reaction with fluorine gas (F₂). Further, 1,1,2,3,4-pentachloropentafluorobutane can be converted into the target product 1,2,3,4-tetrachlorohexafluorobutane by a reaction with chlorine monofluoride gas (ClF). A byproduct in the chlorination step other than the above, 1,1,2,4-tetrachloro-3-butene, can be converted into 1,1,2,3,4,4-hexachlorobutane by a reaction with chlorine gas (Cl₂). Then, 1,1,2,3,4,4-hexachlorobutane can be converted into 1,1,2,3,4,4-hexachlorotetrafluorobutane by a reaction with fluorine gas (F₂). Further, 1,1,2,3,4,4-hexachlorotetrafluorobutane can be converted into the target product 1,2,3,4-tetrachlorohexafluorobutane by a reaction with chlorine monofluoride gas (ClF) via 1,1,2,3,4-pentachloropentafluorobutane. In addition, 1,1,2,3,4-pentachloropentafluorobutane is partially generated as the byproduct in the reaction between 1,1,2,3,4,4-hexachlorobutane and fluorine gas (F₂), but 1,1,2,3,4-pentachloropentafluorobutane can be converted into the target product 1,2,3,4-tetrachlorohexafluorobutane by the reaction with chlorine monofluoride gas (ClF) as noted above. Further, 1,1,2,3,4,4-hexachlorotetrafluorobutane is partially generated as the byproduct in the reaction between 1,2,3,4-tetrachlorobutane and fluorine gas (F₂). That is, the present invention can produce 1,2,3,4-tetrachlorohexafluorobutane as a valuable material by using each of the byproducts in the chlorination step and the fluorination step, which are conventionally separated and then disposed of as waste at a cost, and the present invention has achieved both reduction in environmental burden and improvement in productivity.

Further, the present inventors have focused on the fact that the byproducts in a battery of the processes can be converted into 1,1,2,3,4-pentachloropentafluorobutane or 1,1,2,3,4,4-hexachlorotetrafluorobutane by using reaction reagents (Cl₂ and F₂), which are the same as in the method of reacting 1,3-butadiene and chlorine (Cl₂) to obtain 1,2,3,4-tetrachlorobutane and reacting 1,2,3,4-tetrachlorobutane and fluorine (F₂) to produce 1,2,3,4-tetrachlorohexafluorobutane. Then, the present inventors have made intensive investigation, and consequently found that a liquid containing a battery of the byproducts can be used in the fluorination step without purification of 1,2,3,4-tetrachlorobutane by distillation in the chlorination step. That is, it has been found that, even when distillation facilities are omitted in the chlorination step, highly pure 1,2,3,4-tetrachlorohexafluorobutane can be obtained with common distillation facilities without addition of excessive purification facilities in the next fluorination step. One reason for the fact that the byproducts conventionally disposed can be converted into 1,2,3,4-tetrachlorohexafluorobutane as the valuable material in the present invention is progress of understandings of structure of 1,3,4-trichloro-1-butene, 1,1,4-trichloro-2-butene, 1,1,2,3,4-pentachlorobutane, and 1,1,2,4-tetrachloro-3-butene, which are the byproducts in the step of chlorinating 1,3-butadiene, and each of these can be derived to the valuable material by an appropriate reaction with Cl₂, F₂, or ClF.

Hereinafter, each of the steps will be described.

(Step (a): step of contacting a composition containing at least one selected from group consisting of 1-chloro-1,3-butadiene, 1,3,4-trichloro-1-butene, and 1,1,4-trichloro-2-butene with chlorine (Cl₂) to obtain a composition containing 1,1,2,3,4-pentachlorobutane)

In step (a), a composition containing at least one selected from the group consisting of 1-chloro-1,3-butadiene, 1,3,4-trichloro-1-butene, and 1,1,4-trichloro-2-butene is contacted with chlorine (Cl₂) to obtain a composition containing 1,1,2,3,4-pentachlorobutane. The preferable reaction conditions in step (a) are as follows, for example.
Reaction temperature:
   preferably -20 to 40°C, more preferably -20 to 20°C.
Reaction pressure:
   preferably 0 to 1.0 MPaG, more preferably 0 to 0.1 MPaG. Hereinafter, a gauge pressure is described as "MPaG".

### Reaction solvent:

As specific examples of the preferable solvent, carbon tetrachloride, chloroform, dichloromethane, 1,2,3,4-tetrachlorobutane (dl), 1,2,3,4-tetrachlorohexafluorobutane, and the like can be used. When carbon tetrachloride, chloroform, or dichloromethane is used as the solvent, distillation operation is essential for separating the product and the solvent. Here, 1,2,3,4-tetrachlorobutane has a dl-form and a meso-form. The dl-form has a melting point of -10°C or lower and a boiling point of 213°C, and the meso-form has a melting point of 73°C and a boiling point of 218°C. According to these differences in the physical properties, the dl-form and the meso-form can be separated. In addition, the dl-form can be used as the solvent, and the meso-form cannot be used as the solvent due to these physical properties. Using 1,2,3,4-tetrachlorobutane (dl) as the solvent has an advantage that there is no necessity to separate the product from the solvent. Using 1,2,3,4-tetrachlorohexafluorobutane as the solvent has an advantage that there is no necessity to separate the product from the solvent in the subsequent fluorination reaction step. In addition, the above reaction can be performed without a solvent. In this case, there is an advantage of an efficient reaction with chlorine (Cl₂) gas compared with the case of using the solvent, but the reactivity with chlorine gas (Cl₂) decreases in the latter half of the reaction as the product concentration increases. In addition, for the reaction without a solvent, the reaction solvent in the preceding step of reacting 1,3-butadiene and chlorine (Cl₂); and the product 1,2,3,4-tetrachlorobutane is necessarily separated in advance by distillation. That is, when 1,2,3,4-tetrachlorobutane (dl) or 1,2,3,4-tetrachlorohexafluorobutane is used as the reaction solvent in the preceding step of reacting 1,3-butadiene and chlorine (Cl₂), the crude reaction solution can be used as it is in step (a).

### Raw material:

As the composition containing at least one selected from the group consisting of 1-chloro-1,3-butadiene, 1,3,4-trichloro-1-butene, and 1,1,4-trichloro-2-butene, which is used as the raw material, the byproducts contained in the product in the reaction between 1,3-butadiene and chlorine (Cl₂) is preferably used in terms of effective utilization of the byproducts. The composition containing at least one selected from the group consisting of 1-chloro-1,3-butadiene, 1,3,4-trichloro-1-butene, and 1,1,4-trichloro-2-butene may further contain 1,1,2,4-tetrachloro-3-butene. The product in the reaction between 1,3-butadiene and chlorine (Cl₂) further containing 1,1,2,3,4-pentachlorobutane and increasing the concentration of 1,1,2,3,4-pentachlorobutane in the reaction solution by generating 1,1,2,3,4-pentachlorobutane by step (a) can effectively utilize the byproducts. It is found that 1,2,3,4-tetrachlorobutane generated in the reaction between butadiene and chlorine has a ratio of the dl-from: the meso-form of about 1:3. When the dl-1,2,3,4-tetrachlorobutane is used as the solvent, it is desirably used repeatedly due to the generation ratio, and thus, the meso-1,2,3,4-tetrachlorobutane is desirably separated by crystallization as noted in Examples, described later. When the dl-1,2,3,4-tetrachlorobutane obtained in this crystallization is repeatedly reused as the solvent for the reaction between butadiene and chlorine, 1,3,4-trichloro-1-butene, 1,1,4-trichloro-2-butene, 1,1,2,3,4-pentachlorobutane, and 1,1,2,4-tetrachloro-3-butene are accumulated. As noted in Examples, described later, using the liquid containing these accumulated byproducts as the raw material liquid in step (a) can yield the target product efficiently. The reaction of 1,1,2,4-tetrachloro-3-butene with chlorine in step (a) generates 1,1,2,3,4,4-hexachlorobutane. The reaction of 1,1,2,3,4,4-hexachlorobutane with fluorine in the next step (b) generates 1,1,2,3,4,4-hexachlorotetrafluorobutane, and partially further generates 1,1,2,3,4-pentachloropentafluorobutane. In the reactions of 1,1,2,3,4,4-hexachlorotetrafluorobutane and 1,1,2,3,4-pentachloropentafluorobutane generated here with chlorine monofluoride in the further subsequent step (c), 1,1,2,3,4,4-hexachlorotetrafluorobutane preferentially reacts to generate 1,1,2,3,4-pentachloropentafluorobutane, and then 1,1,2,3,4-pentachloropentafluorobutane reacts to generate the target 1,2,3,4-tetrachlorohexafluorobutane. In the case where the unreacted 1,1,2,4-tetrachloro-3-butene remains in step (a), 1,1,2,4-tetrachlorohexafluorobutane is generated in the next step (b). Since 1,1,2,4-tetrachlorohexafluorobutane and the target 1,2,3,4-tetrachlorohexafluorobutane have a regioisomer relationship and have similar physical property values, it is difficult to separate them. Thus, it is important to perform the reaction in step (a) so that the unreacted 1,1,2,4-tetrachloro-3-butene does not remain. If the unreacted 1,3,4-trichloro-1-butene and 1,1,4-trichloro-2-butene remain in step (a), trichloroheptafluorobutane is generated in step (b), but it can be easily separated by conventionally performed distillation purification with utilizing a difference from boiling point of the target product 1,2,3,4-tetrachlorohexafluorobutane. Note that it is desired that the reaction be performed in step (a) so that the unreacted 1-chloro-1,3-butadiene and 1,3,4-trichloro-1-butene do not remain because the final yield of the target product increases.

### Desired configuration of reaction apparatus:

(i) Examples of a material of the reaction apparatus such as a reaction vessel and piping include alloys such as stainless steel, Inconel, Hastelloy, and Monel, glass, polytetrafluoroethylene (PTFE), perfluoroalkoxyalkane (PFA), glass lining, PFA lining, and PTFE lining, and a reaction vessel made of glass, PTFE, or PFA can be used when the reaction is performed under the atmospheric pressure. It is preferable that the reactor be made of glass, polytetrafluoroethylene (PTFE), perfluoroalkoxyalkane (PFA), glass lining, PFA lining, or PTFE lining that has no risk of elution of a metal chloride in the repeated use.
(ii) The reaction between chlorine (Cl₂) and the composition containing at least one selected from the group consisting of 1-chloro-1,3-butadiene, 1,3,4-trichloro-1-butene, and 1,1,4-trichloro-2-butene is preferably performed by contacting chlorine gas (Cl₂) with a liquid of the composition containing at least one selected from the group consisting of 1-chloro-1,3-butadiene, 1,3,4-trichloro-1-butene, and 1,1,4-trichloro-2-butene by bubbling or the like. The amount of introduced chlorine gas (Cl₂) needs to be greater than or equal to the stoichiometric amount relative to double bonds in the composition containing at least one selected from the group consisting of 1-chloro-1,3-butadiene, 1,3,4-trichloro-1-butene, and 1,1,4-trichloro-2-butene. The amount of introduced chlorine gas (Cl₂) is preferably 1.0 time to 10.0 times, and more preferably 1.0 to 3.0 times of the stoichiometric amount. Using an excess amount of chlorine gas (Cl₂) can reduce the unreacted composition containing at least one selected from the group consisting of 1-chloro-1,3-butadiene, 1,3,4-trichloro-1-butene, and 1,1,4-trichloro-2-butene, but the disposal amount of unreacted chlorine gas (Cl₂) increases, which is not economical. In addition, introducing the excess amount of chlorine (Cl₂) has disadvantages that a load in a step of degassing dissolved chlorine from the reaction solution after the reaction is finished increases, resulting enlargement of the equipment, and the like.
(iii) Due to low reactivity of 1,3,4-trichloro-1-butene, 1,1,4-trichloro-2-butene, and 1,1,2,4-tetrachloro-3-butene with chlorine (Cl₂), contacting them with chlorine gas (Cl₂) by bubbling or the like does not induce the reaction immediately. If chlorine gas (Cl₂) is continuously introduced at a flowrate higher than a consumption rate of chlorine gas (Cl₂) determined by the reaction, chlorine (Cl₂) dissolved in the reaction solution is saturated, and unreacted chlorine (Cl₂) moves to the gas phase, which becomes waste. Thus, it is favorable that the flowrate of chlorine (Cl₂) be regulated while determining consumption of 1,3,4-trichloro-1-butene, 1,1,4-trichloro-2-butene, and 1,1,2,4-tetrachloro-3-butene.
(iv) Due to low reactivity of 1,3,4-trichloro-1-butene, 1,1,4-trichloro-2-butene, and 1,1,2,4-tetrachloro-3-butene with chlorine (Cl₂), irradiation with light having a wavelength shorter than 480 nm (visible ray to ultraviolet ray (UV)) to generate chlorine radicals is effective in order to efficiently complete the reaction. As for a method for installing the light-radiating apparatus, the apparatus may be installed inside the reactor, or may be installed outside the reactor. When the light-radiating apparatus is installed outside the reactor, the reactor may have a window that transmits light to irradiate the inside reaction solution with light, or a pump-circulating piping attached to the reactor may have a window that transmits light to irradiate the reaction solution flowing in the piping with light.

(Step (b): step of contacting the composition containing 1,1,2,3,4-pentachlorobutane obtained in step (a) with fluorine (F₂) to obtain a composition containing 1,1,2,3,4-pentachloropentafluorobutane)

In step (b), the composition containing 1,1,2,3,4-pentachlorobutane obtained in step (a) is contacted with fluorine (F₂) to obtain the composition containing 1,1,2,3,4-pentachloropentafluorobutane. The preferable reaction conditions in step (b) are as follows, for example.

### Reaction temperature:

If the reaction temperature is low, utilizing efficiency of fluorine gas (F₂) decreases. If the reaction temperature is high, a decomposition reaction involved with cleavage of C-C bonds may occur, which causes decrease in the reaction yield. For these reasons, the reaction temperature is set to preferably 0 to 80°C, and more preferably 0 to 40°C. Since the reactivity decreases as the fluorination reaction proceeds, a treatment such that the reaction temperature is raised as the reaction proceeds in the latter half may be performed.

### Reaction pressure:

Fluorine gas (F₂) has high reactivity, and a high pressure is unpreferable for stably continuing the reaction for a long time. Thus, the pressure inside the reactor is set to preferably -0.02 to 0.20 MPaG, and more preferably -0.01 to 0.05 MPaG in terms of gauge pressure.

### Fluorine gas (F₂) concentration:

Fluorine gas (F₂) used in the reaction may be single fluorine gas (F₂), but may be introduced as a diluted mixed gas diluted with inert gas such as nitrogen. If the concentration of fluorine gas (F₂) is high, a decomposition reaction involved with cleavage of C-C bonds may occur, which become a cause of decrease in the reaction yield. Also, in order to stably continue the reaction for a long time, the concentration of fluorine gas (F₂) is favorably regulated to control the reactivity. If the concentration of fluorine gas (F₂) is low, the production efficiency decreases, which is industrially disadvantageous. Thus, the concentration of fluorine gas (F₂) is regulated to preferably 20 to 80 vol%, and more preferably 40 to 70 vol%.

### Reaction solvent:

As the solvent, known solvents that do not react with the raw material can be used. As specific examples of the preferable solvent, tetrachloromethane, hexachloroethane, trichlorotrifluoroethane, tetrachlorodifluoroethane, 1,2,3,4-tetrachlorohexafluorobutane (boiling point: 134°C), and the like can be used. The target product 1,2,3,4-tetrachlorohexafluorobutane (boiling point: 134°C) is desirably used. The reason therefor is that a step of separating the solvent after the reaction can be omitted because the target product is used as the solvent.

### Raw material:

The composition containing 1,1,2,3,4-pentachlorobutane used as the raw material and obtained in step (a) may contain 1,1,2,3,4,4-hexachlorobutane. When the raw material used in step (a) contains 1,1,2,4-tetrachloro-3-butene, 1,1,2,3,4,4-hexachlorobutane is generated from 1,1,2,4-tetrachloro-3-butene via step (a).

### Desired configuration of reaction apparatus:

Examples of a material of the reaction apparatus such as a reaction vessel and piping include alloys such as stainless steel, Monel, Inconel, and Hastelloy, and nickel. As long as the concentration of fluorine gas (F₂) is regulated to 20 vol% or less and the reaction is performed under the atmospheric pressure, a reactor made of PFA or PTFE can also be used.

As for a method for introducing fluorine gas (F₂), utilizing efficiency of fluorine gas (F₂) can be increased by combining common methods such as: a method of connecting a plurality of reactors in series, contacting the composition containing 1,1,2,3,4-pentachlorobutane and fluorine gas (F₂), and introducing unreacted fluorine gas (F₂) discharged from the preceding reactor partially or entirely into the subsequent reactor; and a method of installing a stirring apparatus in the reactor.

The crude 1,1,2,3,4-pentachloropentafluorobutane obtained by the reaction between the composition containing 1,1,2,3,4-pentachlorobutane and fluorine (F₂) can be purified by a common purification operation such as distillation.

(Step (c) step of contacting composition containing 1,1,2,3,4-pentachloropentafluorobutane obtained in step (b) and chlorine monofluoride (ClF) to obtain composition containing 1,2,3,4-tetrachlorohexafluorobutane)
In step (c), the composition containing 1,1,2,3,4-pentachloropentafluorobutane obtained in step (b) is contacted with chlorine monofluoride (ClF) to obtain the composition containing 1,2,3,4-tetrachlorohexafluorobutane. The preferable reaction conditions in step (c) are as follows, for example.

### Reaction temperature:

If the reaction temperature is low, reactivity of chlorine monofluoride (ClF) decreases to fail to utilize chlorine monofluoride (ClF) for the reaction efficiently. On the other hand, if the reaction temperature is high, more energy is required, which is not economical. For these reasons, the reaction temperature is preferably 50°C to 180°C, and more preferably 80°C to 150°C.

### Reaction pressure:

ClF gas (F₂) has high reactivity, and a high pressure is unpreferable for stably continuing the reaction for a long time. Thus, the pressure inside the reactor is set to preferably -0.02 to 0.20 MPaG, and more preferably 0 to 0.10 MPaG in terms of gauge pressure.

### ClF gas concentration:

ClF gas used for the reaction may be single ClF gas, or may be introduced as a diluted mixed gas diluted with an inert gas such as nitrogen. If the concentration of ClF gas is high and a flowrate thereof is also high, an excessive reaction occurs locally to generate trichloroheptafluorobutane, which causes decrease in reaction selectivity. In addition, to stably continue the reaction for a long time, balance between the concentration and flowrate of ClF gas is preferably regulated to control the reactivity. If the concentration of ClF gas is low, the production efficiency decreases, which is industrially disadvantageous. Thus, as for the concentration of ClF gas, balance with the flowrate needs to be considered, and regulated to preferably 20 to 100 vol%, and more preferably 60 to 100 vol%. ClF gas is introduced into the reactor at a flowrate of preferably 1 to 3000 liter/hour (L/h), and more preferably 1 to 1500 L/h. For example, when a 2-L reactor is used on a laboratory scale, ClF gas is introduced at a flowrate of preferably 2 to 60 liter/hour (L/h), and more preferably 6 to 40 L/h. In particular, when the reaction is performed on an industrial scale, a large flowrate of 500 L/h or more is required, although depending on the reactor size. Since the concentration of 1,2,3,4-tetrachlorohexafluorobutane in the reaction solution increases to form conditions such that excess reaction products are easily generated as the reaction proceeds, the flowrate and concentration of ClF gas to be introduced may be appropriately regulated as the reaction proceeds.

### Reaction solvent:

When the reaction is performed in a liquid phase, the target product 1,2,3,4-tetrachlorohexafluorobutane (boiling point: 134°C) is desirably used as the solvent. The first reason therefor is that a step of separating the solvent after the reaction can be omitted because the target product is used as the solvent. The second reason therefor is that the target product 1,2,3,4-tetrachlorohexafluorobutane is considered to be used similarly in the reaction with fluorine (F₂) in the preceding step. In addition, the reaction can be performed without a solvent.

### Raw material:

The composition containing 1,1,2,3,4-pentachloropentafluorobutane obtained in step (b) may contain 1,1,2,3,4,4-hexachlorotetrafluorobutane. When the raw material used in step (a) contains 1,1,2,4-tetrachloro-3-butene, 1,1,2,3,4,4-hexachlorotetrafluorobutane is generated via 1,1,2,3,4,4-hexachlorobutane generated from 1,1,2,4-tetrachloro-3-butene via step (a), and further via step (b). In addition, 1,1,2,3,4,4-hexachlorotetrafluorobutane is partially generated also from 1,1,2,3,4-pentachlorobutane by a side reaction via step (b).

### Desired configuration of reaction apparatus:

(i) Examples of a material of the reaction apparatus such as a reaction vessel and piping include alloys such as stainless steel, Monel, Inconel, and Hastelloy, glass, nickel, polytetrafluoroethylene (PTFE), and perfluoroalkoxyalkane (PFA). When the reaction is performed under the atmospheric pressure, a reaction vessel made of glass or a reactor made of PTFE or PFA can be used.
(ii) As for the method for introducing ClF, the contact efficiency between gas and liquid can be increased by using a common method such as a single-pipe type and an aeration-plate type (a sintered metal filter or a PTFE filter) to decrease a bubble diameter, and utilizing efficiency of ClF can be increased. In addition, the utilizing efficiency of ClF can be increased by combining common methods such as: a method of connecting a plurality of reactors in series, contacting the composition containing 1,1,2,3,4-pentachloropentafluorobutane with ClF gas, and introducing unreacted ClF gas discharged from the preceding reactor partially or entirely into the subsequent reactor; and a method of installing a stirring apparatus in the reactor.
(iii) The reaction can be performed with a batch type, or a continuous type. The batch type allows the reaction conveniently, and the continuous type allows the reaction efficiently.
(iv) The reaction solution (crude 1,2,3,4-tetrachlorohexafluorobutane) obtained by the reaction between the composition containing 1,1,2,3,4-pentachloropentafluorobutane and chlorine monofluoride (ClF) can be purified by a common purification operation such as distillation.
(v) By attaching a condenser that liquefies again vapor of the reaction solution in the latter part of the reactor, loss of the reaction solution can be reduced to increase the production efficiency. In addition, with utilizing a difference in boiling point of the composition containing the raw material 1,1,2,3,4-pentachloropentafluorobutane and 1,2,3,4-tetrachlorohexafluorobutane, the production efficiency can be increased by installing an apparatus that liquefies vapor of 1,2,3,4-tetrachlorohexafluorobutane for recovery in the latter part of the reactor.

### Additive:

Adding hydrogen fluoride (HF) can increase the reactivity of chlorine monofluoride (ClF) and allow the reaction efficiency. It is important to allow HF to be present in the reaction solution, but the reaction solution is heated to a temperature equal to or higher than the boiling point of HF, and thus, HF is continuously vaporized to move to the gas phase portion. Thus, the effect can be efficiently obtained by connecting a cooling condenser to a gas outlet of the reactor to condense HF vapor, and returning HF into the reaction solution again. When HF is vaporized to decrease the HF concentration in the reaction solution, the effect is deteriorated. Thus, it is preferable that HF be appropriately introduced in the middle of the reaction. HF may be continuously introduced simultaneously with ClF, and in this case, the amount of HF to be introduced relative to ClF is regulated to preferably 0.001 to 0.200 mol-times, more preferably 0.001 to 0.160 mol-times, further preferably 0.003 to 0.080 mol-times, and most preferably 0.003 to 0.040 mol-times.

### Others:

The progress of the reaction can be determined by using a common analysis device such as gas chromatography.

### (As for utilization of inert gas)

Examples of the inert gas used in the present invention include nitrogen, helium, neon, and argon. The concentration of the inert gas is as described in each of the above-described sections.

### EXAMPLES

The present invention will be specifically described with the following examples, but the scope of the present invention is not limited to the following examples. The following Examples were performed according to the reaction scheme in [Chemical Formula 1].

### Example 1

### (i) Chlorination reaction of 1,3-butadiene (C₄H₆+2Cl₂ -> C₄H₆Cl₄)

Into a 4-L reactor made of glass equipped with a gas introducing port and a gas outlet to which a -20°C condenser was connected, 3000 g of chloroform (CHCl₃) was fed as a reaction solvent, and the reactor temperature was set to 10°C. Then, while stirring with a magnetic stirrer, 1,3-butadiene gas and chlorine gas (Cl₂) were sparged into the liquid phase portion through the gas introducing port at a flowrate of 11.8 liter/hour (L/hr) and 24.0 L/hr, respectively, under a light-shielded environment, and introducing the gases was terminated after 9.5 hours. After the stirring was continued for 1.0 hour, nitrogen gas was sparged through the gas introducing port to purge chlorine gas (Cl₂) dissolved in the reaction solution, and then the reactor was opened to take out the reaction solution. As a result of analysis of the reaction solution, 1,2,3,4-tetrachlorobutane (C₄H₆Cl₄) was obtained in 87% yield (yield amount: 853 g). As byproducts, 1,3,4-trichloro-1-butene (C₄H₅Cl₃), 1,1,4-trichloro-2-butene (C₄H₅Cl₃), 1,1,2,3,4-pentachlorobutane (C₄H₅Cl₅), and 1,1,2,4-tetrachloro-3-butene (C₄H₄Cl₄) were contained at 2.6 wt% in total (relative to 100 wt% of a weight (3967 g) of the entire solution after the reaction including the weight of the solvent CHCl₃).

By using 3000 g of this reaction solution, the solvent was recovered by distillation (packing: HELI PACK No. 2/HETP: 39 mm, tower height: 100 cm, number of theoretical plates: 25.6) to subsequently obtain 72 g of a low-boiling-point fraction containing 53 wt% of 1,3,4-trichloro-1-butene, 23 wt% of 1,1,4-trichloro-2-butene, and 16 wt% of 1,2,3,4-tetrachlorobutane.

### (ii) Chlorination reaction of 1,3,4-trichloro-1-butene and 1,1,4-trichloro-2-butene (C₄H₅Cl₃+Cl₂ -> C₄H₅Cl₅)

Into a 100-ml reactor made of glass equipped with a gas introducing port and a gas outlet, 60 g of the low-boiling-point fraction containing 53 wt% of 1,3,4-trichloro-1-butene, 23 wt% of 1,1,4-trichloro-2-butene, and 16 wt% of 1,2,3,4-tetrachlorobutane, obtained in Example 1 (i), was fed, and cooled to 0°C. Then, 50-vol% chlorine gas (Cl₂) diluted with nitrogen was sparged into the liquid phase portion at a flowrate of 2.0 liter/hour (L/hr) under a natural-light environment for 10 hours, and then the reaction was terminated. Nitrogen gas was sparged through the gas introducing port to purge chlorine gas (Cl₂) dissolved in the reaction solution, and then the reactor was opened to take out 78 g of the reaction solution. As a result of analysis of this reaction solution, the concentration of 1,1,2,3,4-pentachlorobutane (C₄H₅Cl₅) was 77 wt%, and the concentration of 1,2,3,4-tetrachlorobutane was 12 wt%. The yield of 1,1,2,3,4-pentachlorobutane based on 1,3,4-trichloro-1-butene and 1,1,4-trichloro-2-butene was 92%. Note that 1,2,3,4-tetrachlorobutane did not react.

### (iii) Fluorination reaction of 1,1,2,3,4-pentachlorobutane (C₄H₅Cl₅+5F₂ -> C₄Cl₅F₅+5HF)

Into a 200-ml reactor made of PFA equipped with a gas introducing port and a gas outlet, 62 g of the reaction solution containing 1,1,2,3,4-pentachlorobutane (C₄H₅Cl₅) at a concentration of 77 wt% and 1,2,3,4-tetrachlorobutane at a concentration of 12 wt%, obtained in the procedure of Example 1 (ii), was fed, 145 g of a solvent 1,2,3,4-tetrachlorohexafluorobutane was added, and the mixture was cooled to 0°C. Then, 20-vol% fluorine gas diluted with nitrogen gas (F₂/N₂) was sparged into the liquid phase portion at a flowrate of 14.8 liter/hour (L/hr) for 26 hours, and then the reaction was terminated. The temperature of the reactor was raised to 40°C, nitrogen gas was sparged through the gas introducing port to purge fluorine gas (F₂) dissolved in the reaction solution, and then the reactor was opened to take out 208 g of the reaction solution. As a result of analysis of this reaction solution, the concentration of 1,1,2,3,4-pentachloropentafluorobutane was 29 wt%, and the concentration of 1,2,3,4-tetrachlorohexafluorobutane contained in the reaction solution was 68 wt% including the amount added as the solvent. The yield of 1,1,2,3,4-pentachloropentafluorobutane based on 1,1,2,3,4-pentachlorobutane was 91%.

### (iv) Cl-F exchanging reaction of 1,1,2,3,4-pentachloropentafluorobutane (C₄Cl₅F₅+ClF -> C₄Cl₄F₆+Cl₂)

Into a 200-ml reactor made of PFA equipped with a gas introducing port and a gas outlet, 200 g of the reaction solution containing 1,1,2,3,4-pentachloropentafluorobutane at a concentration of 29 wt%, obtained in Example 1 (iii), was fed, a -20°C cooling condenser was connected on the gas outlet side of the reactor, and the reactor was heated to 130°C. Then, 50-vol% chlorine monofluoride gas (ClF) diluted with nitrogen was sparged at a flowrate of 2.4 liter/hour (L/hr) for 5.5 hours, and then the reaction was terminated. Nitrogen gas was sparged through the gas introducing port to parge chlorine monofluoride gas (ClF) dissolved in the reaction solution, and then the reactor was opened to take out 194 g of the reaction solution. As a result of analysis of this reaction solution, the concentration of 1,2,3,4-tetrachlorohexafluorobutane was 96 wt%. The yield of 1,2,3,4-tetrachlorohexafluorobutane based on 1,1,2,3,4-pentachloropentafluorobutane was 90%.

According to the present invention, the byproducts in each step, which are conventionally wasted as industrial waste, are converted into the target product. The yield of 1,2,3,4-tetrachlorohexafluorobutane was increased by 16% compared with the case where the present invention was not applied.

### Example 2

### (i) Chlorination reaction of 1,3,4-trichloro-1-butene and 1,1,4-trichloro-2-butene (C₄H₅Cl₃+Cl₂ -> C₄H₅Cl₅)

Into a 100-ml reactor made of glass equipped with a gas introducing port and a gas outlet, 60 g of a low-boiling-point fraction containing 53 wt% of 1,3,4-trichloro-1-butene, 23 wt% of 1,1,4-trichloro-2-butene, and 16 wt% of 1,2,3,4-tetrachlorobutane, obtained by the procedure similar to Example 1 (i), was fed, and cooled to 0°C. Then, 50-vol% chlorine gas (Cl₂) diluted with nitrogen was sparged into the liquid phase portion at a flowrate of 2.0 liter/hour (L/hr) under a light-shielded environment for 10 hours, and then the reaction was terminated. Nitrogen gas was sparged through the gas introducing port to purge chlorine (Cl₂) dissolved in the reaction solution, and then the reactor was opened to take out 71 g of the reaction solution. As a result of analysis of this reaction solution, the concentration of 1,1,2,3,4-pentachlorobutane was 55 wt%, and the concentration of 1,2,3,4-tetrachlorobutane was 13 wt%. The concentration of unreacted 1,3,4-trichloro-1-butene was 14 wt%, and the concentration of unreacted 1,1,4-trichloro-2-butene was 11 wt%. The yield of 1,1,2,3,4-pentachlorobutane based on 1,3,4-trichloro-1-butene and 1,1,4-trichloro-2-butene was 60%. Note that 1,2,3,4-tetrachlorobutane did not react.

### (ii) Fluorination reaction of 1,1,2,3,4-pentachlorobutane (C₄H₅Cl₅+5F₂ -> C₄Cl₅F₅+5HF)

Into a 200-ml reactor made of PFA equipped with a gas introducing port and a gas outlet, 64 g of the reaction solution containing 1,1,2,3,4-pentachlorobutane at a concentration of 55 wt% and 1,2,3,4-tetrachlorobutane at a concentration of 13 wt%, obtained in the procedure of Example 2 (i), was fed, 150 g of a solvent 1,2,3,4-tetrachlorohexafluorobutane was added, and the mixture was cooled to 0°C. Then, 20-vol% fluorine gas diluted with nitrogen gas (F₂/N₂) was sparged into the liquid phase portion at a flowrate of 14.8 liter/hour (L/hr) for 31 hours, and then the reaction was terminated. The temperature of the reactor was raised to 40°C, nitrogen gas was sparged through the gas introducing port to purge fluorine gas (F₂) dissolved in the reaction solution, and then the reactor was opened to take out 214 g of the reaction solution. As a result of analysis of this reaction solution, the concentration of 1,1,2,3,4-pentachloropentafluorobutane was 20 wt%, and the concentration of 1,2,3,4-tetrachlorohexafluorobutane contained in the reaction solution was 71 wt% including the amount added as the solvent. The yield of 1,1,2,3,4-pentachloropentafluorobutane based on 1,1,2,3,4-pentachlorobutane was 90%.

According to the present invention, the byproducts in each step, which are conventionally wasted as industrial waste, are converted into the target product. The yield of 1,2,3,4-tetrachlorohexafluorobutane was increased by 11% compared with the case where the present invention was not applied.

### Example 3

Example 3 is an example to show that 1,1,2,4-tetrachloro-3-butene is converted into the target product 1,2,3,4-tetrachlorohexafluorobutane via 1,1,2,3,4,4-hexachlorobutane, 1,1,2,3,4,4-hexachlorotetrafluorobutane, and 1,1,2,3,4-pentachloropentafluorobutane. With a one-pathway reaction, 1,1,2,4-tetrachloro-3-butene exhibits a low generation ratio. Thus, by repeatedly using the solvent for a sufficient concentration to show the process into the target product 1,2,3,4-tetrachlorohexafluorobutane via 1,1,2,3,4,4-hexachlorobutane, 1,1,2,3,4,4-hexachlorotetrafluorobutane, and 1,1,2,3,4-pentachloropentafluorobutane, these were accumulated in the solvent to perform a battery of the reactions.

### (i) Chlorination reaction of 1,3-butadiene (C₄H₆+2Cl₂ -> C₄H₆Cl₄)

Into a 3-L reactor made of glass equipped with a gas introducing port and a gas outlet to which a -20°C condenser was connected, 1600 g of dl-1,2,3,4-tetrachlorobutane was fed as a reaction solvent, and the reactor temperature was set to 10°C. Then, while stirring with a magnetic stirrer, 1,3-butadiene gas and chlorine gas (Cl₂) were sparged through the gas introducing port into the liquid phase portion at a flowrate of 11.8 liter/hour (L/hr) and 24.0 L/hr, respectively, under a light-shielded environment, and introducing the gases was terminated after 9.5 hours. After the stirring was continued for 1.0 hour, nitrogen gas was sparged through the gas introducing port to purge chlorine (Cl₂) dissolved in the reaction solution, and then the reactor was opened to take out the reaction solution. As a result of analysis of the reaction solution, 1,2,3,4-tetrachlorobutane (C₄H₆Cl₄) was obtained in 82% yield (yield amount: 799 g) in which 1,2,3,4-tetrachlorobutane added as the solvent was subtracted.

This reaction solution was cooled to -5°C to precipitate meso-1,2,3,4-tetrachlorobutane, and solid and liquid were separated by centrifugal separation. The obtained solid was meso-1,2,3,4-tetrachlorobutane with purity of 97.9 wt%. On the other hand, the separated liquid contained 1,2,3,4-tetrachlorobutane as the main component, and 1,3,4-trichloro-1-butene, 1,1,4-trichloro-2-butene, 1,1,2,3,4-pentachlorobutane, and 1,1,2,4-tetrachloro-3-butene as byproducts. This separated liquid was recovered, and repeatedly used three times in total as the reaction solvent in the similar reactions.

As a result of analysis of composition of the separated liquid after the third reaction, the concentration of dl-1,2,3,4-tetrachlorobutane was 77.2 wt%, the concentration of meso-1,2,3,4-tetrachlorobutane was 5.3 wt%, the concentration of 1,3,4-trichloro-1-butene was 7.7 wt%, the concentration of 1,1,4-trichloro-2-butene was 3.2 wt%, the concentration of 1,1,2,3,4-pentachlorobutane was 4.0 wt%, and the concentration of 1,1,2,4-tetrachloro-3-butene was 2.6 wt%. This separated liquid was recovered as a solvent, and used for the next chlorination reaction of 1,3-butadiene.

### (ii) Chlorination reaction of 1,3-butadiene (C₄H₆+2Cl₂ -> C₄H₆Cl₄)

Into a 3-L reactor made of glass equipped with a gas introducing port and a gas outlet to which a -20°C condenser was connected, 1600 g of the recovered solvent obtained in Example 3 (i) was fed as a reaction solvent, and the reactor temperature was set to 10°C. Then, while stirring with a magnetic stirrer, 1,3-butadiene gas and chlorine gas (Cl₂) were sparged through the gas introducing port into the liquid phase portion at a flowrate of 11.8 liter/hour (L/hr) and 24.0 L/hr, respectively, under a light-shielded environment, and introducing the gases was terminated after 9.5 hours. After the stirring was continued for 1.0 hour, nitrogen gas was sparged through the gas introducing port to purge chlorine (Cl₂) dissolved in the reaction solution, and then the reactor was opened to take out the reaction solution. As a result of analysis of the reaction solution, 1,2,3,4-tetrachlorobutane was obtained in 80% yield (yield amount: 780 g) in which 1,2,3,4-tetrachlorobutane added as the solvent was subtracted.

This reaction solution was cooled to -5°C to precipitate meso-1,2,3,4-tetrachlorobutane, and solid and liquid were separated by centrifugal separation. The obtained solid was meso-1,2,3,4-tetrachlorobutane with purity of 98.2 wt%. On the other hand, as a result of analysis of composition of the separated liquid, the concentration of dl-1,2,3,4-tetrachlorobutane was 71.7 wt%, the concentration of meso-1,2,3,4-tetrachlorobutane was 5.3 wt%, the concentration of 1,3,4-trichloro-1-butene was 9.7 wt%, the concentration of 1,1,4-trichloro-2-butene was 4.1 wt%, the concentration of 1,1,2,3,4-pentachlorobutane was 5.3 wt%, and the concentration of 1,1,2,4-tetrachloro-3-butene was 3.1 wt%.

### (iii) Chlorination reaction of 1,3,4-trichloro-1-butene, 1,1,4-trichloro-2-butene, and 1,1,2,4-tetrachloro-3-butene (C₄H₅Cl₃+Cl₂ -> C₄H₅Cl₅ / C₄H₄Cl₄+Cl₂ -> C₄H₄Cl₆)

Into a 3-L reactor made of glass equipped with a gas introducing port and a gas outlet, 1900 g of the separated liquid containing dl-1,2,3,4-tetrachlorobutane at a concentration of 71.7 wt%, meso-1,2,3,4-tetrachlorobutane at a concentration of 5.3 wt%, 1,3,4-trichloro-1-butene at a concentration of 9.7 wt%, 1,1,4-trichloro-2-butene at a concentration of 4.1 wt%, 1,1,2,3,4-pentachlorobutane at a concentration of 5.3 wt%, and 1,1,2,4-tetrachloro-3-butene at a concentration of 3.1 wt%, obtained in Example 3 (ii), was fed, and heated to 20°C. Then, the liquid was irradiated with UV at a wavelength of 385 nm with an LED-type UV radiation apparatus, 50-vol% chlorine gas (Cl₂) diluted with nitrogen was sparged into the liquid phase portion at a flowrate of 25.0 liter/hour (L/hr) for 6.5 hours, and then introducing chlorine gas (Cl₂) was terminated. The reaction solution was further continuously stirred for 2 hours, and then UV irradiation was terminated. Then, nitrogen gas was sparged through the gas introducing port to purge chlorine (Cl₂) dissolved in the reaction solution, and then the reactor was opened to take out 1988 g of the reaction solution. As a result of analysis of this reaction solution, the concentration of dl-1,2,3,4-tetrachlorobutane was 67.2 wt%, the concentration of meso-1,2,3,4-tetrachlorobutane was 5.1 wt%, the concentration of 1,3,4-trichloro-1-butene was 0.2 wt%, the concentration of 1,1,4-trichloro-2-butene was 0.1 wt%, the concentration of 1,1,2,3,4-pentachlorobutane was 22.6 wt%, the concentration of 1,1,2,4-tetrachloro-3-butene was 0 wt%, and the concentration of 1,1,2,3,4,4-hexachlorobutane was 4.0 wt%. The yield of 1,1,2,3,4-pentachlorobutane based on 1,3,4-trichloro-1-butene and 1,1,4-trichloro-2-butene was 92%. The yield of 1,1,2,3,4,4-hexachlorobutane based on 1,1,2,4-tetrachloro-3-butene was 99%.

### (iv) Fluorination reaction of 1,1,2,3,4-pentachlorobutane and 1,1,2,3,4,4-hexachlorobutane (C₄H₅Cl₅+5F₂ -> C₄Cl₅F₅+5HF, C₄H₄Cl₆+4F₂ -> C₄Cl₆F₄+4HF)

Into a 8-L vertical reactor made of SUS equipped with a gas introducing port and a gas outlet and with a jacket through which cooling water flowed, 1800 g of the reaction solution containing dl-1,2,3,4-tetrachlorobutane at a concentration of 67.2 wt%, meso-1,2,3,4-tetrachlorobutane at a concentration of 5.1 wt%, 1,3,4-trichloro-1-butene at a concentration of 0.2 wt%, 1,1,4-trichloro-2-butene at a concentration of 0.1 wt%, 1,1,2,3,4-pentachlorobutane at a concentration of 22.6 wt%, 1,1,2,4-tetrachloro-3-butene at a concentration of 0 wt%, and 1,1,2,3,4,4-hexachlorobutane at a concentration of 4.0 wt%, obtained by the procedure of Example 3 (iii), was fed, 4200 g of a solvent 1,2,3,4-tetrachlorohexafluorobutane was added, a -20°C cooling condenser was connected on the gas outlet side of the reactor, and the reactor was cooled to 10°C. Then, 20-vol% fluorine gas diluted with nitrogen gas (F₂/N₂) was sparged and introduced into the liquid phase portion at a flowrate of 444 liter/hour (L/hr) for 12 hours. Thereafter, the diluted fluorine gas (F₂/N₂) was regulated to a concentration of 33 vol% and a flowrate of 538 L/hr, and the reaction was continued for 4 hours. After the reaction was terminated, the temperature of the reactor was raised to 40°C, nitrogen gas was sparged through the gas introducing port to purge fluorine gas (F₂) dissolved in the reaction solution, and then the reactor was opened to take out 6822 g of the reaction solution. As a result of analysis of this reaction solution, the concentration of 1,1,2,3,4-pentachloropentafluorobutane was 10.4 wt%, the concentration of 1,1,2,3,4,4-hexachlorotetrafluorobutane was 1.4 wt%, and the concentration of 1,2,3,4-tetrachlorohexafluorobutane contained in the reaction solution was 85.7 wt% including the amount added as the solvent. The yield of 1,1,2,3,4-pentachloropentafluorobutane based on 1,1,2,3,4-pentachlorobutane was 125%. The reason why the yield of 1,1,2,3,4-pentachloropentafluorobutane exceeded 100% was that 1,1,2,3,4-pentachloropentafluorobutane included one generated from 1,1,2,3,4-pentachlorobutane and one generated in side reactions from 1,2,3,4-tetrachlorobutane and 1,1,2,3,4,4-hexachlorobutane. The yield of 1,1,2,3,4,4-hexachlorotetrafluorobutane based on 1,1,2,3,4,4-hexachlorobutane was 104%. The reason why the yield of 1,1,2,3,4,4-hexachlorotetrafluorobutane exceeded 100% was that 1,1,2,3,4,4-hexachlorotetrafluorobutane included one generated from 1,1,2,3,4,4-hexachlorobutane and one generated in side reactions from 1,2,3,4-tetrachlorobutane and 1,1,2,3,4-pentachlorobutane.

### (v) Cl-F exchanging reaction of 1,1,2,3,4-pentachloropentafluorobutane and 1,1,2,3,4,4-hexachlorotetrafluorobutane (C₄Cl₅F₅+ClF -> C₄Cl₄F₆+Cl₂, C₄Cl₆F₄+2ClF -> C₄Cl₄F₆+2Cl₂)

Into a 2-L vertical reactor made of SUS equipped with a gas introducing port and a gas outlet and with a jacket through which a heating medium flowed, 2000 g of the reaction solution containing 1,1,2,3,4-pentachloropentafluorobutane and 1,1,2,3,4,4-hexachlorotetrafluorobutane at a concentration of 10.4 wt% and 1.4 wt%, respectively, and 1,2,3,4-tetrachlorohexafluorobutane at 85.7 wt% including the amount added as the solvent, obtained in Example 3 (iv), was fed, a -30°C cooling condenser was connected on the gas outlet side of the reactor, and the reactor was heated to 110°C. Then, chlorine monofluoride gas (ClF) was sparged and introduced at a flowrate of 11.5 liter/hour (L/hr) for 10 minutes. Heating the reactor was terminated, nitrogen gas was sparged through the gas introducing port to purge chlorine monofluoride gas (ClF) dissolved in the reaction solution, and then a sample of the reaction solution was taken. As a result of analysis of this reaction solution, the concentration of 1,1,2,3,4-pentachloropentafluorobutane was 10.8 wt%, and the concentration of 1,1,2,3,4,4-hexachlorotetrafluorobutane was 0.4 wt%. The reactor was heated again to 110°C, chlorine monofluoride gas (ClF) was sparged and introduced at a flowrate of 11.5 L/hr for 140 minutes, and then the reaction was terminated. Heating the reactor was terminated, nitrogen gas was sparged through the gas introducing port to purge chlorine monofluoride gas (ClF) dissolved in the reaction solution, and then the reactor was opened to take out 1960 g of the reaction solution. As a result of analysis of this reaction solution, the concentration of 1,1,2,3,4-pentachloropentafluorobutane was 1.4 wt%, the concentration of 1,1,2,3,4,4-hexachlorotetrafluorobutane was 0 wt%, the concentration of 1,2,3,4-tetrachlorohexafluorobutane contained in the reaction solution was 96.5 wt% including the amount added as the solvent. The yield of 1,2,3,4-tetrachlorohexafluorobutane based on 1,1,2,3,4-pentachloropentafluorobutane was 90%. The ClF utilizing efficiency (= the reaction consumption amount of ClF / the introduced amount of ClF × 100) was 57%.

According to the present invention, the byproducts in each step, which are conventionally wasted as industrial waste, are converted into the target product. The yield of 1,2,3,4-tetrachlorohexafluorobutane was increased by 23% compared with the case where the present invention was not applied.

### Example 4

### Cl-F exchanging reaction of 1,1,2,3,4-pentachloropentafluorobutane and 1,1,2,3,4,4-hexachlorotetrafluorobutane (C₄Cl₅F₅+ClF -> C₄Cl₄F₆+Cl₂, C₄Cl₆F₄+2ClF -> C₄Cl₄F₆+2Cl₂)

The reaction was performed under entirely the same condition as in Example 3 except for the introducing time of ClF gas and adding hydrogen fluoride (HF) in the reaction conditions of the Cl-F exchanging reaction described in Example 3.

Hydrogen fluoride was added by a method of mixing with chlorine monofluoride gas (ClF) to be continuously introduced. A mixed gas composed of 97-vol% chlorine monofluoride gas (ClF) and 3-vol% hydrogen fluoride gas was sparged and introduced at a flowrate of 11.9 liter/hour (L/hr) for 10 minutes. Heating the reactor was terminated, nitrogen gas was sparged through the gas introducing port to purge chlorine monofluoride gas (ClF) dissolved in the reaction solution, and then a sample of the reaction solution was taken. As a result of analysis of this reaction solution, the concentration of 1,1,2,3,4-pentachloropentafluorobutane was 11.2 wt%, and the concentration of 1,1,2,3,4,4-hexachlorotetrafluorobutane was 0.1 wt%. The reactor was heated again to 110°C, the mixed gas composed of 97-vol% chlorine monofluoride gas (ClF) and 3-vol% hydrogen fluoride (HF) gas was sparged and introduced at a flowrate of 11.9 L/hr for 120 minutes, and then the reaction was terminated. Heating the reactor was terminated, nitrogen gas was sparged through the gas introducing port to purge chlorine monofluoride gas (ClF) dissolved in the reaction solution, and then the reactor was opened to take out 1965 g of the reaction solution. As a result of analysis of this reaction solution, the concentration of 1,1,2,3,4-pentachloropentafluorobutane was 1.0 wt%, the concentration of 1,1,2,3,4,4-hexachlorotetrafluorobutane was 0 wt%, and the concentration of 1,2,3,4-tetrachlorohexafluorobutane contained in the reaction solution was 96.7 wt% including the amount added as the solvent. The yield of 1,2,3,4-tetrachlorohexafluorobutane based on 1,1,2,3,4-pentachloropentafluorobutane was 94%. The ClF utilizing efficiency (= the reaction consumption amount of ClF / the introduced amount of ClF × 100) was 68%.

According to the present invention, the byproducts in each step, which are conventionally wasted as industrial waste, are converted into the target product. The yield of 1,2,3,4-tetrachlorohexafluorobutane was increased by 24% compared with the case where the present invention was not applied.

### Example 5

### Chlorination reaction of 1,3-butadiene (C₄H₆+2Cl₂ -> C₄H₆Cl₄)

Into a 4-L reactor made of glass equipped with a gas introducing port and a gas outlet to which a -20°C condenser was connected, 3000 g of chloroform was fed as a reaction solvent, and the reactor temperature was set to 0°C. Then, while stirring with a magnetic stirrer, 1,3-butadiene gas and chlorine gas (Cl₂) were sparged into the liquid phase portion through the gas introducing port at a flowrate of 11.8 liter/hour (L/hr) and 11.8 L/hr, respectively, under a light-shielded environment, the reaction was terminated after 9.5 hours, and a sample of a part of the reaction solution was taken. As a result of analysis of the collected reaction solution, it was determined that 1-chloro-1,3-butadiene (C₄H₅Cl) was generated as a byproduct. Thereafter, chlorine gas (Cl₂) was further sparged into the liquid phase portion through the gas introducing port at a flowrate of 11.8 L/hr, the reaction was terminated after 9.5 hours, nitrogen gas was sparged through the gas introducing port to purge chlorine (Cl₂) dissolved in the reaction solution, and then the reactor was opened to take out the reaction solution. As a result of analysis of the reaction solution, 1,2,3,4-tetrachlorobutane was obtained in 83% yield (yield amount: 824 g). As byproducts, 1,3,4-trichloro-1-butene, 1,1,4-trichloro-2-butene, 1,1,2,3,4-pentachlorobutane, and 1,1,2,4-tetrachloro-3-butene were contained at 1.8 wt%, 0.7 wt%, 0.9 wt%, and 0.2 wt% (relative to 100 wt% of the weight of the entire solution after the reaction including the weight of the solvent CHCl₃), respectively. In addition, 1-chloro-1,3-butadiene was consumed in the reaction, and was not contained in the liquid after the reaction was terminated.

According to Example 5, it was found that 1-chloro-1,3-butadiene was generated in the middle of the chlorination reaction of 1,3-butadiene, but further chlorinated to 1,3,4-trichloro-1-butene and 1,1,2,3,4-pentachlorobutane.

### INDUSTRIAL APPLICABILITY

In the method for producing 1,2,3,4-tetrachlorohexafluorobutane from 1,3-butadiene as the starting material, the byproducts and the like generated by the chlorination reaction of 1,3-butadiene can be converted into 1,2,3,4-tetrachlorohexafluorobutane as the valuable material to increase the productivity of 1,2,3,4-tetrachlorohexafluorobutane.

## Claims

1. A method for producing 1,1,2,3,4-pentachloro-1,2,3,4,4-pentafluorobutane, the method comprising:
(a) a step of contacting a composition containing at least one selected from the group consisting of 1-chloro-1,3-butadiene, 1,3,4-trichloro-1-butene, and 1,1,4-trichloro-2-butene with chlorine (Cl₂) to obtain a composition containing 1,1,2,3,4-pentachlorobutane; and
(b) a step of contacting the composition containing 1,1,2,3,4-pentachlorobutane obtained in step (a) with fluorine (F₂) to obtain a composition containing 1,1,2,3,4-pentachloro-1,2,3,4,4-pentafluorobutane.

2. The method according to claim 1, wherein the composition containing at least one selected from the group consisting of 1-chloro-1,3-butadiene, 1,3,4-trichloro-1-butene, and 1,1,4-trichloro-2-butene in step (a) contains a product of a reaction between 1,3-butadiene and chlorine (Cl₂).

3. The method according to claim 2, wherein the product of the reaction between 1,3-butadiene and chlorine (Cl₂) further contains 1,1,2,3,4-pentachlorobutane, and generating 1,1,2,3,4-pentachlorobutane by step (a) increases a concentration of 1,1,2,3,4-pentachlorobutane in a reaction solution.

4. The method according to claim 2 or 3, wherein the product of the reaction between 1,3-butadiene and chlorine (Cl₂) further contains 1,1,2,4-tetrachloro-3-butene.

5. The method according to any one of claims 1 to 3, wherein UV irradiation is performed in step (a).

6. A method for producing 1,2,3,4-tetrachloro-1,1,2,3,4,4-hexafluorobutane, the method comprising:
(a) a step of contacting a composition containing at least one selected from the group consisting of 1-chloro-1,3-butadiene, 1,3,4-trichloro-1-butene, and 1,1,4-trichloro-2-butene with chlorine (Cl₂) to obtain a composition containing 1,1,2,3,4-pentachlorobutane;
(b) a step of contacting the composition containing 1,1,2,3,4-pentachlorobutane obtained in step (a) with fluorine (F₂) to obtain a composition containing 1,1,2,3,4-pentachloro-1,2,3,4,4-pentafluorobutane; and
(c) a step of contacting the composition containing 1,1,2,3,4-pentachloro-1,2,3,4,4-pentafluorobutane obtained in step (b) with chlorine monofluoride (ClF) to obtain a composition containing 1,2,3,4-tetrachloro-1,1,2,3,4,4-hexafluorobutane.

7. The method according to claim 6, wherein the composition containing at least one selected from the group consisting of 1-chloro-1,3-butadiene, 1,3,4-trichloro-1-butene, and 1,1,4-trichloro-2-butene in step (a) contains a product of a reaction between 1,3-butadiene and chlorine (Cl₂).

8. The method according to claim 7, wherein the product of the reaction between 1,3-butadiene and chlorine (Cl₂) further contains 1,1,2,3,4-pentachlorobutane, and generating 1,1,2,3,4-pentachlorobutane by step (a) increases a concentration of 1,1,2,3,4-pentachlorobutane in a reaction solution.

9. The method according to claim 7 or 8, wherein the product of the reaction between 1,3-butadiene and chlorine (Cl₂) further contains 1,1,2,4-tetrachloro-3-butene.

10. The method according to any one of claims 6 to 8, wherein UV irradiation is performed in step (a).
